**Europäisches Patentamt**

**European Patent Office**

**Office européen des brevets**

(11) Veröffentlichungsnummer: **0 212 642**
**A1**

(12) # EUROPÄISCHE PATENTANMELDUNG

(21) Anmeldenummer: **86111647.3**

(22) Anmeldetag: **22.08.86**

(51) Int. Cl.⁴: **G 01 N 33/52, G 01 N 31/22,
A 61 J 3/06**

(30) Priorität: **28.08.85 DE 3530621**

(43) Veröffentlichungstag der Anmeldung: **04.03.87
Patentblatt 87/10**

(84) Benannte Vertragsstaaten: **AT BE CH DE FR GB IT LI LU
NL SE**

(71) Anmelder: **Boehringer Mannheim GmbH, Sandhofer
Strasse 116, D-6800 Mannheim 31 (DE)**

(72) Erfinder: **Korb, Heinz, Pillauerstrasse 8,
D-6944 Hemsbach (DE)**
Erfinder: **Lessmann, Hans-Dieter, Dr. rer. nat.,
Mörikestrasse 3 d, D-6945 Hirschberg-Grosssachsen
(DE)**
Erfinder: **Portenhauser, Rudolf, Dr. rer. nat.,
Kustermannstrasse 26 b, D-8132 Tutzing (DE)**

(54) **Verfahren zur Herstellung von präzisen Dosiseinheiten eines Reagenzbestandteiles.**

(57) Die Erfindung betrifft ein Verfahren zum Herstellen von
präzisen Dosiseinheiten von Reagenzbestandteilen, wobei
der in einer Trägerflüssigkeit gelöste Reagenzbestandteil
auf einen saugfähigen, in der Trägerflüssigkeit wenig löslichen Trägerkörper aufgebracht und das Lösungsmittel entfernt wird.

EP 0 212 642 A1

## Verfahren zur Herstellung von präzisen Dosiseinheiten eines Reagenzbestandteiles

Die Erfindung betrifft ein Verfahren zum Herstellen von trockenen, lagerbeständigen, tablettenartigen präzisen Dosiseinheiten eines Reagenzbestandteiles zur Auflösung in einer unvollständigen Reagenzlösung zur Bildung eines flüssigen Reagenz, dessen Reaktion mit einer Probe zu einer analytischen Bestimmung ausgewertet wird, insbesondere für Testpackungen zur diagnostischen Untersuchung von Körperflüssigkeiten.

In der modernen Analytik, insbesondere bei der sogenannten klinisch-chemischen Analyse von Körperflüssigkeiten des Menschen oder der Tiere werden vielfach Fertigreagenzien verwendet, die mehrere Reagenzbestandteile in flüssiger Form enthalten. Derartige flüssige Reagenzien sind einfach zu handhaben und eignen sich besonders für die Automatisierung der Untersuchungen. Dem Anwender wird nach Möglichkeit eine einzige fertige Reagenzlösung für die Bestimmung eines bestimmten Bestandteiles der Körperflüssigkeiten (eines sogenannten Parameters) zur Verfügung gestellt.

Dies ist jedoch nicht immer möglich. In vielen Fällen muß das fertige Reagenz Bestandteile enthalten, die zusammen mit den übrigen Bestandteilen der Reagenzlösung in flüssiger Form nicht ausreichend lagerfähig sind. Dies kann beispielsweise darauf zurückzuführen sein, daß eine unerwünschte Reaktion zwischen den verschiedenen Reagenzbestandteilen abläuft oder daß der betreffende Reagenzbestandteil grundsäztlich nicht in flüssiger Form lagerfähig ist. Derartige Bestandteile werde im folgenden auch als lösungsinstabile Bestandteile bezeichnet.

In derartigen Fällen ist es notwendig oder zumindest wünschenswert,. daß einzelne Bestandteile eines flüssigen Reagenz, wie sie insbesondere für Testpackungen zur diagnostischen Untersuchung von Körperflüssigkeiten verwendet werden, in trockener Form als lagerbeständige Dosiseinheiten zur Verfügung gestellt werden müssen. Hierzu wurden bereits verschieden Formen von Reagenzträgern vorgeschlagen. So wurden lösungsinstabile Reagenzbestandteile beispielsweise auf Papiere oder Vliese imprägniert. Diese werden in Stücke passender Größe zerschnitten, um die entsprechenden Dosiseinheiten zu bilden. Die Stücke werden in die unvollständige Reagenzlösung, die die übrigen Reagenzbestandteile enthält, gegeben und von dem Papier eluiert.

Ein anderes Beispiel von Dosiseinheiten von Reagenzbestandteilen in trockener Form sind Reagenztabletten. Diese werden konventionell in ähnlicher Weise wie Tabletten für therapeutische Anwendungen hergestellt. Hierzu wird der Reagenzbestandteil mit entsprechenden Hilfs- und Trägerstoffen gemischt, homogenisiert und schließlich zu Einzeltabletten verpreßt. Diese Reagenztabletten haben sich für eine Vielzahl diagnostischer Anwendungen bewährt. Sie eignen sich jedoch nicht für Fälle, in denen die Dosiseinheiten des Reagenzbestandteils sehr präzise sein müssen. Die Präzision der Dosierung eines Bestandteils einer in üblicher Weise hergestellten Tablette hängt einerseits von der Homogenität des Gemischs vor der Tablettierung und andererseits von der Präzision des Tablettierungsvorgangs, also dem Tablettengewicht ab. Selbst mit hohem Aufwand ergeben sich dabei Schwankungen des Gehalts an einem bestimmten Bestandteil von Tablette zu Tablette von mindestens 2 %. Die Präzision ist für eine Reihe von klinisch chemischen Reagenzien nicht ausreichend.

Dies gilt insbesondere für Kontrollseren. Mit dem Begriff
Kontrollserum bzw. allgemeine Kontrollmaterialien werden
Eichflüssigkeiten für die klinische Chemie bezeichnet, die
bestimmte zu untersuchende Substanzen (Parameter) in
hochpräziser Dosierung enthalten. Sie dienen der Prüfung und
Eichung der klinisch-chemischen Diagnoseverfahren. Häufig sind
die fraglichen Bestandteile im flüssigen Zustand unbeständig
und müssen daher in trockener Form in den Testpackungen
enthalten sein.

Für derartige Zwecke werden häufig Lyophilisate verwendet.
Diese werden üblicherweise hergestellt, indem man in kleine
Flaschen eine den entsprechenden Bestandteil enthaltende Lösung
präzise einpipettiert und den Inhalt lyophilisiert. Die
Flaschen mit dem Lyophilisat sind in den Testpackungen
enthalten. Erst beim Anwender wird die Reagenzlösung, die
insbesondere ein Kontrollserum sein kann, durch Einfüllen einer
präzisen Menge an Rekonstitutionsflüssigkeit rekonstituiert.
Die Genauigkeit der Dosierung des fraglichen
Reagenzbestandteils in der fertigen Reagenzlösung ist in diesem
Fall sowohl von der Präszision des Pipettiervorgangs beim
Hersteller vor der Lyophilisierung als auch von der Präzision
des Pipettiervorgangs beim Anwender zum Zwecke der
Rekonstitution der Flüssigkeit abhängig. Der letztgenannte
Pipettiervorgang stellt häufig eine Fehlerquelle dar. Selbst
wenn aber beim Anwender ausreichend präzise Dosiergeräte
verwendet werden und mit hoher Sorgfalt gearbeitet wird, ist
der Vorgang zumindest sehr aufwendig.

Aufgabe der vorliegenden Erfindung ist es daher, eine
Dosiseinheit eines Reagenzbestandteils für ein flüssiges
Reagenz in trockener und damit lagerfähiger Form zur Verfügung
zu stellen, die einfach zu handhaben ist und unter Vermeidung
von Fehlerquellen, die beim Anwender des Reagenz entstehen
können, höchste Genauigkeit garantiert.

Diese Aufgabe wird bei einem Verfahren der eingangs gekennzeichneten Art dadurch gelöst, daß der Reagenzbestandteil in einer Trägerflüssigkeit homogen verteilt wird, um eine Auftropfflüssigkeit zu bilden, daß ein für die Auftropfflüssigkeit saugfähiger Trägerkörper zur Verfügung gestellt wird, der aus einem in der Testflüssigkeit zerfallenden und sich darin derartig lösenden oder dispergierenden Material besteht, daß er die Auswertung der Reaktion nicht stört, wobei das Material des Trägerkörpers und das Lösungsmittel so aufeinander abgestimmt sind, daß die Lösung den Trägerkörper nicht so stark anlöst, daß er seine Form wesentlich ändert, daß die Auftropfflüssigkeit auf den Trägerkörper so aufgebracht wird, daß dieser sie aufsaugt und daß der Trägerkörper nach dem Aufbringen der Auftropfflüssigkeit einem Trocknungsvorgang unterzogen wird.

Die Präzision der so hergestellten Dosiseinheit entspricht im wesentlichen der Präzision des Auftropfvorgangs. Ein maximaler Fehler von 0,5 % ist erfindungsgemäß ohne weiteres zu erreichen.

Der Reagenzbestandteil kann irgendein, insbesondere lösungsinstabiler Bestandteil eines Reagenz für Zwecke der klinischen Chemie sein, beispielsweise eine niedermolekulare oder hochmolekulare organische Verbindung, ein Enzym oder ein immunologischer Reagenzbestandteil. Der Begriff Reagenz im Sinne der vorliegenden Erfindung umfaßt auch die oben erwähnten Kontrollseren auf die sich die Erfindung besonders richtet. In diesem Fall ist der Reagenzbestandteil die in den Körperflüssigkeiten zu bestimmende Substanz. Der Begriff unvollständige Reagenzlösung soll ganz allgemein die Flüssigkeit bezeichnen, in die die erfindungsgemäß hergestellte Dosiseinheit gegeben wird, um ein flüssiges Reagenz herzustellen. Die unvollständige Reagenzlösung kann deshalb ein reines Lösungsmittel sein, in der Regel wird sie jedoch bereits Substanzen enthalten, die für die Reaktion zur analytischen Bestimmung einer Probe notwendig sind.

Das durch Auflösung der tablettenartigen Dosiseinheit in der unvollständigen Reagenzlösung gebildete Reagenz wird üblicherweise mit der Probe vermischt, wobei sich nach einer Inkubationszeit ein Farbumschlag einstellt, der photometrisch ausgewertet wird. Selbstverständlich kann die Zugabe weiterer Reagenzien oder die Durchführung weiterer Behandlungsschritte notwendig oder zweckmäßig sein.

Die Erfindung basiert wesentlich auf der Erkenntnis, daß das Prinzip der konventionellen Tablettierung, nämlich die Dosierung der Tablettenbestandteile an einer relativ großen Menge von Tablettenmasse einmal vorzunehmen, diese Tablettenmasse dann zu homogenisieren und daraus die Dosiseinheiten unter Vermeidung einzelner Dosierungsvorgänge zu gleichgroßen Tabletten zu verpressen, dem Erreichen einer sehr hohen Dosispräzision im Wege steht. Entgegen diesen Grundsätzen der Tablettierung schlägt die Erfindung vor, die Dosis eines Tablettenbestandteils, auf dessen Präzision es besonders ankommt, in Form eines Auftropfvorgangs aufzubringen.

Zu diesem Zweck wird der betreffende Reagenzbestandteil in einer Trägerflüssigkeit homogen verteilt. Dies kann in suspendierter oder in gelöster Form geschehen. Auch eine Emulsion kann verwendet werden.

Es kommt für die Erfindung wesentlich darauf an, daß der Trägerkörper, die Trägerflüssigkeit und die Verfahrensbedingungen in Anbetracht der schwierigen Randbedingungen, die bei Reagenzien für die klinische Chemie zu beachten sind, sorgfältig aufeinander abgestimmt werden. Im einzelnen sind dabei folgende Punkte zu beachten, die unabhängig voneinander für die Erfindung wesentlich sind:

a) Der Trägerkörper muß für die Auftropfflüssigkeit so
saugfähig sein, daß diese in ihn eindringt, so daß ihre
Konzentration an der Oberfläche nach dem Aufsaugvorgang
nicht wesentlich größer ist als in den tieferliegenden
Schichten. Je nach der Relation der Menge
Auftropfflüssigkeit zur Größe des Trägerkörpers wird die
Auftropfflüssigkeit sich nicht im ganzen Trägerkörper
verteilen. Dort, wo sie eingedrungen ist, sollte die
Konzentration des lösungsinstabilen Reagenzbestandteils
jedoch weitgehend gleichmäßig sein. Zumindest ist eine
Anreicherung des Reagenzbestandteils an der Oberfläche des
Trägerkörpers zu vermeiden. Dadurch würde nämlich die
Gefahr erhöht, daß schon ein geringer Abrieb an der
Oberfläche der fertigen Dosiseinheit zu einer
Beeinträchtigung der Dosispräzision führt. Für den Fall,
daß die Auftropfflüssigkeit eine Suspension ist, müssen die
Poren des Trägerkörpers deswegen größer sein als die
suspendierten Teilchen. Bevorzugt ist der
Reagenzbestandteil in der Trägerflüssigkeit echt gelöst.
Diese Lösung zieht besonders gut und gleichmäßig in den
Trägerkörper ein.

b) Der Trägerkörper muß aus einem Material bestehen, das
gegenüber der Testfunktion, also bezüglich der Reaktion zur
Bestimmung des gesuchten klinisch-chemischen Parameters und
bezüglich der Auswertung dieser Reaktion, inert ist. Diese
Bedingung ist in Anbetracht der extremen Empfindlichkeit
insbesondere der enzymatischen Reaktionen schwierig zu
erfüllen, beispielsweise wegen der Gefahr der Inhibition
der enzymatischen Aktivität.

c) Das Material des Trägerkörpers darf die Auswertung des Tests nicht stören. Wie erwähnt werden die Reaktionen in der klinischen Chemie in der Regel photometrisch ausgewertet. In diesem Fall sollte sich das Material des Trägerkörpers in der unvollständigen Reagenzlösung optisch klar lösen. Bei anderen Tests wird die Trübung der Flüssigkeit (Turbidimetrie) oder das von dieser ausgehende Streulicht (Nephelometrie) bestimmt. Wieder andere Tests basieren auf der Fluorometrie. Auch in diesen Fällen muß der Trägerkörper aus solchem Material bestehen, daß er, wenn er in der unvollständigen Reagenzlösung zerfallen ist, das Meßergebnis nicht stört.

d) Die Trägerflüssigkeit muß in vielen Fällen eine hohe Konzentration des zu dosierenden lösungsinstabilen Reagenzbestandteils aufnehmen können, um die notwendige Menge in Anbetracht des beschränkten Saugvermögens des Trägerkörpers dosieren zu können. Für den Fall, daß die Trägerflüssigkeit den Reagenzbestandteil löst, muß diese ein gutes Lösungsmittel für diesen Bestandteil sein.

e) Andererseits darf der Auftropfvorgang die mechanische Stabilität des Trägerkörpers nicht beeinträchtigen. Insbesondere darf der Trägerkörper durch die Auftropfflüssigkeit nicht so stark angelöst werden, daß er seine Form so stark ändert, daß er nicht mehr zu gleichgroßen Dosiseinheiten getrocknet werden kann. Dies steht in vielen Fällen im Gegensatz zu der zuvor genannten Bedingung. Gemäß bevorzugter Ausführungsform der Erfindung kann es zweckmäßig sein, daß die Trägerflüssigkeit solche Bestandteile des Trägerkörpers, die sie lösen könnte, in einer Konzentration nahe der Sättigung enthält.

f) Die Verfahrensbedingungen des Trocknungsvorgangs müssen so
gewählt sein, daß die in vielen Fällen nicht gut
wärmebeständigen Reagenzbestandteile nicht beeinträchtigt
werden. Aus diesem Grund ist es in der Regel nicht möglich,
als Trägerflüssigkeit eine Schmelze eines normalerweise bei
Raumtemperatur festen Stoffes zu verwenden, die einfach auf
den Trägerkörper aufgetropft und so weit abgekühlt wird,
daß sie erstarrt. Vielmehr wird nach dem Auftropfen der
Auftropfflüssigkeit die Trocknung bevorzugt dadurch
erreicht, daß die Trägerflüssigkeit verdampft wird, während
der lösungsinstabile Reagenzbestandteil trocken auf dem
Trägerkörper zurückbleibt. Dabei muß es vermieden werden,
daß bei dem Verdampfungsvorgang eine die Präzision der
Dosierung beeinträchtigende Menge des präzise zu
dosierenden lösungsinstabilen Reagenzbestandteils mit
verdampft wird. Quantitativ ausgedrückt muß der Dampfdruck
der Trägerflüssigkeit bei den Bedingungen des
Trocknungsvorgangs um so viel größer sein als der
Reagenzbestandteils, wie es der geforderten Genauigkeit
entspricht. Ist er beispielsweise 1000 mal so groß, so
verdampft bei dem Trocknungsvorgang 1/1000 des zu
dosierenden Reagenzbestandteils. Absolut gesehen muß die
Trägerflüssigkeit so flüchtig sein, daß sie bei
Temperaturen, die die Substanzen auf den Dosiseinheiten
vertragen können, in einem Zeitraum verdampft werden kann,
der eine wirtschaftliche Herstellung der Dosiseinheiten
erlaubt.


Gemäß einer bevorzugten Ausführungsform des Verfahrens wird der
Trocknungsvorgang im Vakuum durchgeführt, um eine schnelle
Trocknung bei vergleichsweise geringen Temperaturen zu
erreichen.

- 9 -     0 212 642

Besonders bevorzugt wird der Trägerkörper nach dem Auftropfen der Auftropflösung so weit abgekühlt, daß die Auftropflösung erstarrt. Dadurch wird der Trägerkörper mit der Auftropflösung mechanisch stabil genug, daß er einer separaten Vakuumtrocknungseinrichtung zugeführt werden kann, in der er im Vakuum getrocknet wird.

Eine bevorzugte Ausführungsform der Erfindung und die damit erzielbaren Vorteile werden im folgenden anhand der Figuren näher erläutert. Es zeigen:

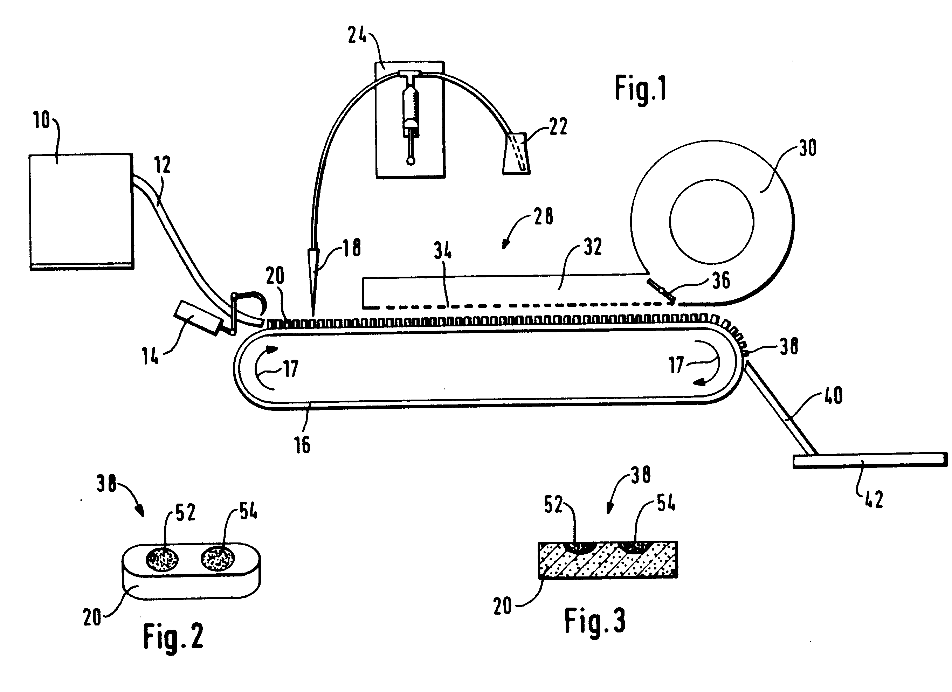

Figur 1 eine schematisierte Seitenansicht einer Vorrichtung zur Durchführung des erfindungsgemäßen Verfahrens,
Figur 2 eine perspektivische Ansicht einer nach dem erfindungsgemäßen Verfahren hergestellten Dosiseinheit,
Figur 3 einen Querschnit durch die Dosiseinheit nach Figur II.

In Figur I erkennt man einen Vorratsbehälter 10 für Trägerkörper. Diese werden in konventioneller Weise über ein Zuführungsrohr 12 und eine Vereinzelungseinrichtung 14 einem Transportband 16 so zugeführt, daß sie auf dem Transportband 16 in äquidistanten Abständen angeordnet sind. Das Transportband 16 bewegt sich schrittweise in Schritten, die dem Tablettenabstand entsprechen, in Richtung der Pfeile 17.

Durch die Bewegung des Transportbandes 16 gelangen die tablettenförmigen Trägerkörper 20 zu der Auftropfstation 18. Dort werden sie mit einer Auftropfflüssigkeit betropft, die einem Vorratsbehälter 22 entnommen wird und mit Hilfe eines Dosiergerätes 24 sehr präzise dosiert werden kann. Das Dosiergerät 24 ist konventionell aufgebaut. Verwendet werden kann beispielsweise ein im klinisch chemischen Labor übliches Pipettiergerät. Es können auch mehrere Auftropfstationen vorgesehen sein, sei es um mehrere lösungsinstabile Bestandteile getrennt aufzutropfen (vgl. Fig II und III) oder um die gleiche Stelle des Trägerkörpers in mehreren Einzelschritten zu betropfen.

Nach dem Auftropfen gelangen die betropften Trägerkörper 20 in den insgesamt mit 28 bezeichneten Trocknungsbereich. Im einfachsten Fall wird zum Trocknen warme Luft verwendet, die von der Gebläse- und Erhitzungseinrichtung 30 erzeugt und in einen Luftschacht 32 getrieben wird, aus dem sie durch Austrittsöffnungen 34 nach unten auf die betropften Trägerkörper 20 auftritt. Die Luftströmung läßt sich über eine Klappe 36 steuern.

Die Länge des Trocknungsbereiches 28 und die Transportgeschwindigkeit des Transportbandes 16 sind so aufeinander abgestimmt, daß am Ende des Trocknungsbereiches eine ausreichende Trocknung der betropften Trägerkörper 20 erreicht ist. Die fertigen erfindungsgemäßen Dosiseinheiten 38 werden über eine Rutsche 40 einem Sammelgefäß 42 zugeführt.

Gemäß einer bevorzugten Ausführungsform der Erfindung kann das Gebläse 30 auch zum Transport von Kühlluft ausgelegt sein. Mit Hilfe des dann aus den Luftaustrittsöffnungen 34 ausströmenden Kühlluftstromes werden die betropften Trägerkörper 20 auf eine Temperatur abgekühlt, die unterhalb des Erstarrungspunktes der an der Auftropfstation 18 aufgetropften Auftropflösung liegt. Bei dieser Version gelangen nicht fertige Dosiseinheiten, sondern die durch das Erstarren verfestigten betropften Trägerkörper in das Sammelgefäß 42. Sie können dann zu geeigneten Zeitpunkten in eine Vakuumtrocknungseinrichtung transportiert werden, um sie im Vakuum unter möglichst geringer Temperaturbeanspruchung zu trocknen.

Bei einem vollkontinuierlichen Verfahren kann es auch vorgesehen sein, daß oberhalb des Transportbandes 16 mit Hilfe geeigneter Schleuseneinrichtung ein evakuierbarer Bereich angeordnet ist, in dem eine Vakuumtrocknung durchgeführt wird.

Die Figuren II und III zeigen beispielhaft erfindungsgemäß hergestellte Dosiseinheiten. In diesem Fall sind die Trägerkörper oval ausgeformt, was besonders günstig ist, wenn mehr als ein Reagenzbestandteil auf einen einzigen Trägerkörper aufgetropft werden soll.

Im dargestellten Fall befinden sich in dem Trägerkörper 20 zwei imprägnierte Bereiche 52 und 54, die zwei verschiedene, miteinander nicht beständige Reagenzbestandteile enthalten. Diese werden gleichzeitig oder nacheinander auf den Trägerkörper 20 aufgetropft, wobei darauf zu achten ist, daß das Saugvermögen des Trägerkörpers 20 und die Menge der Auftropfflüssigkeit so aufeinander abgestimmt sind, daß die imprägnierten Bereiche 52 und 54 voneinander getrennt sind, wie dies in der Figur III gut zu erkennen ist.

Aus dieser Figur erkennt man auch, daß die Auftropfflüssigkeit nur so weit in Tabletten eindringt, wie dies ihrer Menge in Relation zum Saugvermögen der Trägerkörper 20 entspricht. Innerhalb eines imprägnierten Bereiches ist die Konzentration des Reagenzbestandteiles aber weitgehend gleichmäßig.

Durch die folgenden Beispiele wird die Erfindung näher erläutert.

Als geeignete Trägerkörper haben sich insbesondere Trägertabletten aus D-Mannit und Natriumchlorid erwiesen. Diese können beispielsweise wie folgt hergestellt werden.

## A) Herstellung von D-Mannit Trägertabletten

D-Mannit wird nach üblichen Verfahren durch Zugabe von Wasser und anschließender Trocknung granuliert. Dem getrockneten und gesiebten D-Mannit-Granulat werden als Gleit- und Formentrennmittel 3 % (w/w) Polyäthylenglykol 6000 zugemischt. Die so hergestellte Tablettenmasse wird auf üblichen Tablettenmaschinen zu Tabletten verpreßt.

Beschreibung der Tabletten

| | |
|---|---|
| Format: | Rund, biplan |
| | Durchmesser 7 mm, Höhe 3,5 mm |
| Härte: | 22 N (nach Schleuninger) |
| Gewicht: | 140 mg |
| Zusammensetzung: | |
| D-Mannit | 135,800 mg |
| Polyäthylenglykol | 4,200 mg |
| | 140,000 mg |

## B) Herstellung von Natriumchlorid-Trägertabletten

Natriumchlorid wird gemahlen und mit 1 % (w/w) Poly (1-vinyl-2-pyrrolidon) 25000 als Granulierhilfsmittel gemischt. Diese Mischung wird nach üblichem Verfahren durch Zugabe von Wasser und anschließender Trocknung granuliert.
Das getrocknete und gesiebte Granulat wird auf üblichen Tablettenmaschinen zu Tabletten verpreßt.

Beschreibung der Tabletten

| | |
|---|---|
| Format: | rund, biplan |
| | Durchmesser 7 mm, Höhe 3,0 mm |
| Härte: | 24 N (nach Schleuninger) |
| Gewicht: | 178 mg |
| Zusammensetzung: | |
| Natriumchlorid | 176,220 mg |
| Poly (1-vinyl-2-pyrrolidon) 25000 | 1,780 mg |
| | 178,000 mg |

Beispiele erfindungsgemäß hergestellter Dosiseinheiten:

Beispiel 1

Herstellung von Kalium/Calcium /Eisen-Tabletten

1,864 g    Kaliumchlorid
4,838 g    Calciumchlorid  .  2 $H_2O$
0,229 g    Eisen-III-chlorid  .  6 $H_2O$

werden in 10 ml bidest. Wasser gelöst.
Je 20 /ul dieser Lösung werden auf jede gemäß A hergestellte
D-Mannit-Trägertablette aufpipettiert. Die beladenen Tabletten
werden im Vakuum getrocknet.

Beispiel 2

Herstellung von Bilirubin-Tabletten

0,400 g    Bilirubin
0,040 g    Progallin
0,200 g    Tris(hydroxymethyl)aminomethan

werden in 20 ml Dimethylsulfoxid gelöst. Das
Progallin dient als Stabilisator, das
Tris(hydroxymethyl)aminomethan hebt den pH an und erhöht
dadurch die Löslichkeit.
Je 20 /ul dieser Lösung werden auf jede gemäß B hergestellte
Natriumchlorid-Trägertablette aufpipettiert. Die beladenen
Tabletten werden im Vakuum getrocknet.

Beispiel 3

Herstellung von Gamma-GT/$\alpha$-Amylase-Tabletten

0,136 g    Kaliumdihydrogenphosphat
2,100 g    L-(+)-Mononatriumaspartat
0,400 mg   Edetinsäure, Dinatriumsalz  .  2 $H_2O$

werden in 10 ml Wasser gelöst und die Lösung mit verdünnter Natronlauge auf pH 7,5 eingestellt. Das Kaliumdihydrogenphosphat dient als Puffer, das L-(+)-Mono-natriumaspartat und das Edetinsäure, Dinatriumsalz  .  2 $H_2O$ dienen als Stabilisator. In dieser Lösung werden 1,400 kU   Gamma-Glutamyltransferase
2,000 kU   $\alpha$-Amylase
           gelöst.

Je 10 $\mu$l Enzymlösung werden auf jede nach A hergestellte D-Mannit-Trägertablette aufpipettiert. Die beladenen Tabletten werden im Vakuum getrocknet.

Die folgende Tabelle gibt die Variationskoeffizienten des jeweiligen Gehalts der Parameter von Tablette zu Tablette für die beschriebenen Beispiele bei einem Kollektiv von jeweils 20 Tabletten an:

| Beispiel Nr. | Parameter | Variationskoeffizient % |
|---|---|---|
| 1 | Kalium | 0,88 |
| 1 | Calcium | 0,32 |
| 1 | Eisen | 1,16 |
| 2 | Bilirubin | 0,65 |
| 3 | $\gamma$-GT | 0,11 |
| 3 | $\alpha$-Amylase | 0,44 |

Patentansprüche

1. Verfahren zum Herstellen von trockenen, lagerbeständigen, tablettenartigen präzisen Dosiseinheiten eines Reagenzbestandteils zur Auflösung in einer unvollständigen Reagenzlösung zur Bildung eines flüssigen Reagenz, dessen Reaktion mit einer Probe zu einer analytischen Bestimmung ausgewertet wird, insbesondere für Testpackungen zur diagnostischen Untersuchung von Körperflüssigkeiten, dadurch gekennzeichnet,

a)  daß der Reagenzbestandteil in einer Trägerflüssigkeit homogen verteilt wird, um eine Auftropfflüssigkeit zu bilden,

b)  daß ein für die die Auftropfflüssigkeit saugfähiger Trägerkörper zur Verfügung gestellt wird, der aus einem in der unvollständigen Reagenzlösung zerfallenden und sich darin derartig lösenden oder dispergierenden Material besteht, daß er die Auswertung der Reaktion nicht stört,

c)  wobei das Material des Trägerkörpers und das Lösungsmittel so aufeinander abgestimmt sind, daß die Lösung den Trägerkörper nicht so stark anlöst, daß er seine Form wesentlich ändert,

d)  daß die Auftropfflüssigkeit auf den Trägerkörper so aufgebracht wird, daß dieser sie aufsaugt.

e)  und daß der Trägerkörper nach dem Aufbringen der Auftropfflüssigkeit einem Trocknungsvorgang unterzogen wird.

2. Verfahren nach Anspruch 1, dadurch gekennzeichnet, daß der Reagenzbestandteil in der Trägerflüssigkeit gelöst wird, wobei als Trägerflüssigkeit ein Lösungsmittel für den Reagenzbestandteil verwendet wird, das bei Raumtemperatur mindestens einen 100 mal so hohen Dampfdruck wie der Reagenzbestandteil hat, und daß bei dem Trocknungsvorgang das Lösungsmittel entfernt wird.

3. Verfahren nach Anspruch 1, dadurch gekennzeichnet, daß der Reagenzbestandteil in der Trägerflüssigkeit suspendiert wird und der saugfähige Trägerkörper Poren hat, die größer sind als die suspendierten Teilchen.

4. Verfahren nach einem der vorhergehenden Ansprüche, dadurch gekennzeichnet, daß der Trägerkörper Mannit enthält.

5. Verfahren nach einem der vorhergehenden Ansprüche, dadurch gekennzeichnet, daß der Trägerkörper Bestandteile des flüssigen Reagenz enthält.

6. Verfahren nach einem der vorhergehenden Ansprüche, dadurch gekennzeichnet, daß der Trocknungsvorgang zumindest teilweise im Vakuum durchgeführt wird.

7. Verfahren nach Anspruch 6, dadurch gekennzeichnet, daß der Trocknungsvorgang einen Verfahrensschritt einschließt, bei dem der Trägerkörper auf eine Temperatur unterhalb des Erstarrungspunktes der Auftropflösung abgekühlt wird und anschließend bei der gleichen oder einer höheren Temperatur eine Trocknung im Vakuum vorgenommen wird.

　　　　　　　**0 212 642**

8.　Testpackung mit einem Reagenziensatz zur analytischen
　　Untersuchung von Proben, insbesondere von Körperflüssigkeiten, enthaltend
　　eine unvollständige Reagenzlösung, welcher ein Bestandteil, der mit der unvollständigen Reagenzlösung nicht
　　in gelöster Form lagerfähig ist, fehlt und
　　eine Dosiseinheit nach einem der vorhergehenden An-
　　sprüche.

1/1

Fig.1

Fig.2

Fig.3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | EP-A-0 017 414 (FMC CORP.)<br><br>* Insgesamt * | 1,3,5, 6 | G 01 N 33/52<br>G 01 N 31/22<br>A 61 J 3/06 |
| Y |  | 4 |  |
| A |  | 8 |  |
|  | --- |  |  |
| Y | US-A-3 816 262 (A.A. MONTE et al.)<br>* Zusammenfassung; Spalte 1, Zeile 1 - Spalte 11, Zeile 61 * | 4 |  |
| A |  | 1 |  |
|  | --- |  | **RECHERCHIERTE SACHGEBIETE (Int. Cl.4)** |
| A | DE-A-2 857 197 (BOEHRINGER MANNHEIM GmbH)<br>* Seite 6, Zeile 9 - Seite 8, Zeile 11; Ansprüche 1,3-6 * | 1,5,6 | G 01 N<br>A 61 J<br>A 61 K |
|  | --- |  |  |
| A | FR-A-2 169 639 (F. HOFFMANN-LA ROCHE & CIE. S.A.)<br>* Seite 1, Zeile 1 - Seite 3, Zeile 19; Ansprüche 1,2,4,8 * | 1,5 |  |
|  | --- |  |  |
| A | EP-A-0 078 225 (R. HENRY)<br>* Zusammenfassung; Seite 2, Zeile 32 - Seite 4, Zeile 11; Ansprüche 1,2 * | 1 |  |
|  | ----- |  |  |

Der vorliegende Recherchenbericht wurde für alle Patentansprüche erstellt.

| Recherchenort | Abschlußdatum der Recherche | Prüfer |
|---|---|---|
| DEN HAAG | 24-09-1986 | HITCHEN C.E. |